(19) Europäisches Patentamt
European Patent Office
Office européen des brevets

(11) Veröffentlichungsnummer: **0 005 483**
**A1**

(12) # EUROPÄISCHE PATENTANMELDUNG

(21) Anmeldenummer: **79101360.0**

(22) Anmeldetag: **04.05.79**

(51) Int. Cl.²: **C 07 G 7/00**
A 61 K 35/16, A 61 K 37/04

(30) Priorität: **08.05.78 DE 2819969**

(43) Veröffentlichungstag der Anmeldung:
**28.11.79 Patentblatt 79/24**

(84) Benannte Vertragsstaaten:
**AT CH DE FR IT**

(71) Anmelder: **BEHRINGWERKE AKTIENGESELLSCHAFT**
**Postfach 1140**
**D-3550 Marburg/Lahn(DE)**

(72) Erfinder: **Eckert, Theodor, Prof. Dr.**
**Birkenweg 45**
**D-4400 Münster(DE)**

(72) Erfinder: **Bischoff, Heinz-Robert**
**Volkersheimerstieg 14**
**D-3205 Bockenem(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Verfahren zur Anreicherung und Stabilisierung von antihämophilem Globulin (Faktor VIII) und die Verwendung des Verfahrensproduktes.**

(57) Verfahren zur Anreicherung und Stabilisierung von Antihämophilem Globulin durch Fällung des Antihämophilen Globulin mit einer Lösung von Hydroxyäthylstärke sowie die Verwendung des Verfahrensproduktes als Arzneimittel.

EP 0 005 483 A1

Croydon Printing Company Ltd.

HOECHST AKTIENGESELLSCHAFT HOE 78/B 022 Dr.D/Pa

# Verfahren zur Anreicherung und Stabilisierung von Antihämophilem Globulin (Faktor VIII) und die Verwendung des Verfahrensproduktes.

Die Erfindung betrifft ein Verfahren zur Anreicherung und Stabilisierung von Antihämophilem Globulin (Faktor VIII) aus Blutplasma oder Plasmafraktion sowie die Verwendung des Verfahrensproduktes als intravenös applizierbares Arzneimittel.

Der Zweck der Erfindung liegt darin, das in normalem Blutplasma vorliegende Antihämophile Globulin durch einen Fällungsvorgang mit Hydroxyäthylstärke anzureichern und zu stabilisieren.

Zur Anreicherung und Stabilisierung von Antihämophilem Globulin sind bereits andere Verfahren vielfach in Gebrauch, wie beispielsweise Fällungen mit anorganischen Elektrolytsalzen.
Aber auch rein physikalische Verfahren, wie etwa das Verfahren der Kryopräcipitation, bei dem es zu einer Ausfällung des Antihämophilen Globulin durch Kälteeinwirkung kommt, werden häufig angewendet. Darüber hinaus sind Verfahren beschrieben, bei denen es mit Hilfe höhermolekularer Fällungsmittel, wie etwa Polyäthylenglykole oder Dextrane, zu einer mehr oder weniger spezifischen Anreicherung des Antihämophilen Globulin kommt.

Diese Verfahren weisen jedoch alle mehr oder weniger Nachteile auf. So ist beispielsweise die Fällung mit anorganischen Elektrolytsalz nur durch eine relativ geringe Selektivität und eine geringe Stabilität der Fällungsprodukte gekennzeichnet.
Auch das Verfahren der Kryopräcipitation weist Nachteile insbesondere in Bezug auf die Ausbeute und den Reinheitsgrad des zu erzielenden Produktes auf.
Die Fällungen mit Makromolekülen, wie Polyäthylenglykole

oder Dextrane, sind in Bezug auf Ausbeute, Stabilität und Reinheitsgrad des Fällungsproduktes der Präcipitation mit Hydroxyäthylstärke unterlegen.
Auch die physiologische Verträglichkeit der Fällungsprodukte mit Polyäthylenglykolen und Dextranen ist im Vergleich mit den entsprechenden Fällungsprodukten des Antihämophilen Globulin mit Hydroxyäthylstärke nicht so gut.

Der Erfindung liegt die Aufgabe zugrunde, die durch die bisher verwendeten Fällungsmittel zur Anreicherung und Stabilisierung von Antihämophilem Globulin auftretenden Nachteile zu vermindern.

Diese Aufgabe wird erfindungsgemäß dadurch gelöst, daß zur Fällung des Antihämophilen Globulin aus Plasma oder Plasmafraktion Hydroxyäthylstärke, vorzugsweise bei tieferen Temperaturen, verwendet wird.

Die Erfindung betrifft daher ein Verfahren zur Anreicherung und Stabilisierung von Antihämophilem Globulin (Faktor VIII) aus Blutplasma oder Plasmafraktionen, das dadurch gekennzeichnet ist, daß zur Fällung des Antihämophilen Globulin eine Lösung von Hydroxyäthylstärke als Präcipitationsmittel verwendet wird.

Die Hydroxyäthylstärke, die bei dem erfindungsgemäßen Verfahren eingesetzt wird, hat folgende Formel:

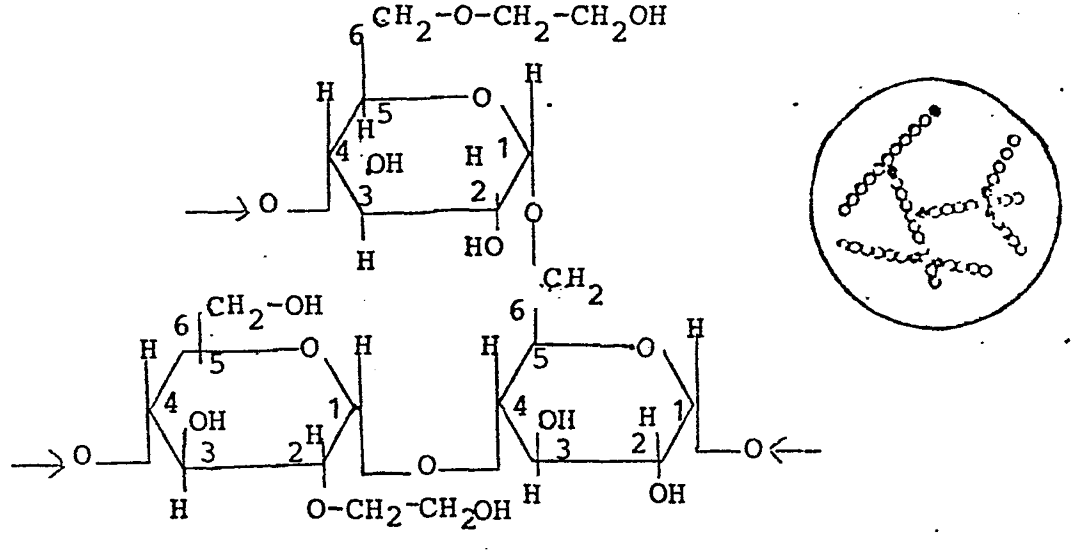

Bei dem erfindungsgemäßen Verfahren wird vorzugsweise einer wäßrigen Lösung von Blutplasma oder Plasmafraktionen eine solche Menge Hydroxyäthylstärke, vorzugsweise in wäßriger Lösung, zugegeben, daß die Faktor VIII-Aktivität präcipitiert. Das Präcipitat wird abgetrennt und in üblicher Weise zu einem intravenös applizierbaren Arzneimittel aufgearbeitet.

Die Erfindung betrifft ferner die Verwendung des Verfahrensproduktes als intravenös applizierbares Arzneimittel.

Die mit der Erfindung erzielten Vorteile bestehen insbesondere darin, daß die Ausbeute, die Stabilität und die Reinheit des auf diese Weise gefällten Antihämophilen Globulin gegenüber den bisher bekannten Fällungsmitteln überraschenderweise wesentlich besser ist.

Das folgende Ausführungsbeispiel erläutert die Erfindung:

Frisches menschliches Plasma, bzw. aufgetautes frisches Tiefkühlplasma, wird bei einer Temperatur von +4$^{o}$C mit einer 10 %igen wäßrigen Lösung von Hydroxyäthylstärke im Verhältnis 1:1 (Plasma : HÄS-Lsg.) gut vermischt und das Lösungsgemisch bei +4$^{o}$C vier Stunden stehen gelassen. Das ausgefallene Präcipitat, das das Antihämophile Globulin enthält, wird weiter aufgearbeitet.

Patentansprüche:

1. Verfahren zur Anreicherung und Stabilisierung von Antihämophilem Globulin (Faktor VIII) aus Blutplasma oder Plasmafraktionen, dadurch gekennzeichnet, daß zur Fällung des Antihämophilen Globulin eine Lösung von Hydroxyäthylstärke als Präcipitationsmitten verwendet wird.

2. Verwendung des nach Anspruch 1 erhaltenen Produktes als intravenös applizierbares Arzneimittel.

0005483

Europäisches Patentamt

# EUROPÄISCHER RECHERCHENBERICHT

Nummer der Anmeldung

EP 79 10 1360

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| A | US - A - 3 758 382 (CHARLES T. KNORPP)<br>* Anspruch 1 *<br>-- | 1 | C 07 G 67/00<br>A 61 K 35/16<br>37/04 |
| A | CHEMICAL ABSTRACTS, Vol. 81, 1974 Nr. 74477e, Nr. 13, 30. September Columbus, Ohio, U.S.A.<br>S. LIEBE et al.: "Cryoprecipitation and dextran precipitation in the production of factor VIII concentrates. Comparative studies on protein composition", Seite 184<br>& Z. Gesamte Inn. Med. Ihre Grenzgeb. 1974, 29(6), 232-6<br>* Zusammenfassung *<br>---- | 1 | RECHERCHIERTE SACHGEBIETE (Int. Cl.²)<br>C 07 G 7/00<br>A 61 K 35/14<br>37/04 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 17-08-1979 | RIJCKEBOSCH |

EPA form 1503.1 06.78